(19) Europäisches Patentamt
European Patent Office
Office européen des brevets

(11) Veröffentlichungsnummer : **0 152 779 B1**

(12) # EUROPÄISCHE PATENTSCHRIFT

(45) Veröffentlichungstag der Patentschrift : **15.06.88**

(51) Int. Cl.⁴ : **C 07 H 15/04**, B 01 J 23/40, B 01 J 23/74

(21) Anmeldenummer : **85100572.8**

(22) Anmeldetag : **21.01.85**

(54) **Verfahren zur Herstellung eines Gemisches von alpha-D-Gluco-pyranosido-1,6-mannit und alpha-D-Glucopyranosido-1,6-sorbit aus alpha-D-Glucopyranosido-1,6-fructose.**

(30) Priorität : **04.02.84 DE 3403973**

(43) Veröffentlichungstag der Anmeldung : **28.08.85 Patentblatt 85/35**

(45) Bekanntmachung des Hinweises auf die Patenterteilung : **15.06.88 Patentblatt 88/24**

(84) Benannte Vertragsstaaten : **AT BE CH DE FR GB IT LI NL SE**

(56) Entgegenhaltungen :
**FR-A- 1 584 796**
**FR-A- 2 310 354**
**GB-A- 2 097 390**
**US-A- 4 018 835**
**US-A- 4 380 680**

(73) Patentinhaber : **BAYER AG**
**Konzernverwaltung RP Patentabteilung**
**D-5090 Leverkusen 1 Bayerwerk (DE)**

(72) Erfinder : **Darsow, Gerhard, Dr.**
**Zu den Tannen 39**
**D-4150 Krefeld (DE)**
Erfinder : **Biedermann, Wolfgang, Dr.**
**Am Heckerhof 49**
**D-4150 Krefeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung eines Gemisches der diastereomeren Zuckeralkohole α-D-Glucopyranosido-1,6-mannit und α-D-Glucopyranosido-1,6-sorbit aus α-D-Glucopyranosido-1,6-fructose durch Hydrierung mit Wasserstoff.

Der Reaktionsverlauf läßt sich durch das folgende Reaktionsschema veranschaulichen :

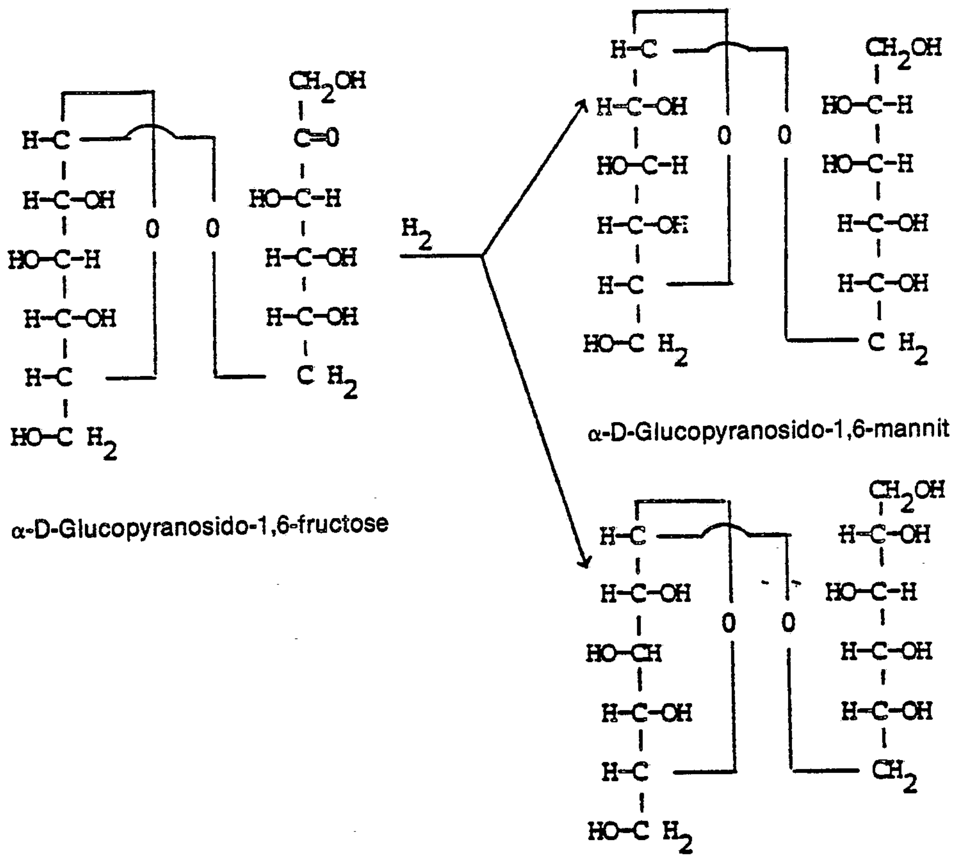


α-D-Glucopyranosido-1,6-fructose

α-D-Glucopyranosido-1,6-mannit (GPM)

α-D-Glucopyranosido-1,6-sorbit (GPS)

Gegenstand der Erfindung ist ein Verfahren zur Herstellung eines Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100-500 bar und Temperaturen von 70-115 °C im Festbettverfahren über als Hydrierungskatalysatoren dienenden trägerfreien Formkörpern mit einer Druckfestigkeit von 120-170 $kg/cm^2$ und einer inneren Oberfläche zwischen 25 und 75 $m^2/g$ von Elementen der 8. Nebengruppe des Periodensystems durchführt.

Es wurde gefunden, daß man die Gemische der Zuckeralkohole α-D-Glucopyranosido-1,6-mannit und α-D-Glucopyranosido-1,6-sorbit in nahezu quantitativer Ausbeute erhält, wenn man die Hydrierung der α-D-Glucopyranosido-1,6-fructose unter den obigen speziellen Reaktionsbedingungen kontinuierlich über als Hydrierungskatalysatoren dienenden trägerfreien Formkörpern von Elementen der 8. Nebengruppe des Periodensystems, insbesondere Nickel, Kobalt und Eisen, durchführt. Dies ist von umso größerer Bedeutung, wenn man bedenkt, daß die Entfernung höhermolekukarer oder niedermolekularer störender Verunreinigungen aus dem Reaktionsgemisch besonders dann einen sehr hohen technischen Aufwand erfordert, wenn man die gewünschten Zuckeralkohole nicht einzeln aus dem Reaktionsgemisch isolieren, sondern durch Einengen ihrer wäßrigen Lösung als Zuckeralkoholgemisch in fester Form gewinnen will.

Aus der GB-A 2 097 390 ist ein Verfahren zur katalytischen Hydrierung von Monosacchariden bekannt. Nach diesem Verfahren können jedoch Disaccharide nicht zu Disaccharidalkoholen hydriert werden, da die Ätherbindung hydrogenolytisch gespalten wird und entsprechende Monosaccharidalkohole entstehen.

Gemäß US-P 4 018 835 ist ein Katalysator bekannt, der aus einer Legierung von Aluminium, Nickel und Eisen gewonnen wird, indem das Aluminium in alkalischer Lösung aus dieser Legierung heraus

gelöst wird. Derartige Katalysatoren führen beim Versuch der Hydrierung von Disacchariden ebenfalls zu Monosaccharidalkoholen und sind somit zur Herstellung von Disaccharidalkoholen aus Disacchariden nicht anwendbar.

Bei den bekannten Verfahren zur Herstellung von α-D-Glucopyranosido-1,6-sorbit (DPS 2 217 628) und α-D-Glucopyranosido-1,6-mannit (DAS 2 520 173) wird im diskontinuierlichen Suspensionsverfahren (Batch-process) als Hydrierkatalysator jeweils ein pulverförmiger Nickelkatalysator eingesetzt. Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch. Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, den pulverförmigen Katalysator gezielt zu dotieren, umzupumpen und quantitativ vom Reaktionsprodukt abzufiltrieren. Die Katalysatorschlammpumpen unterliegen einem hohen mechanischen Verschleiß. Die quantitative Entfernung des pulverförmigen Katalysators aus dem Reaktionsprodukt ist aufwendig. Ferner ist die Gefahr groß, die Katalysatoraktivität durch die zusätzlichen Operationen verhälnismäßig schnell zu verringern. Es ist daher vorteilhaft, die Reaktion über einen fest angeordneten Katalysator ablaufen zu lassen. Ein solcher Katalysator müßte eine hohe Aktivität besitzen, die über einen längeren Zeitraum nicht nachlassen dürfte, weil häufige Katalysatorwechsel bei Festbettreaktionen ebenfalls aufwendig sind.

Alle diese Nachteile werden durch das erfindungsgemäße Verfahren überwunden.

Nach dem erfindungsgemäßen Verfahren ist es möglich, das kristallisierte Gemisch der beiden diastereomeren Zuckeralkohole in einer Reinheit von über 99 % herzustellen, wobei der Gehalt an nicht umgesetzter α-D-Glucopyranosido-1,6-fructose unter 0,1 % und die Summe von Sorbit und Mannit unter 0,2 % liegt. Als Ausgangsverbindung für das erfindungsgemäße Verfahren wird reine kristalline α-D-Glucopyranosido-1,6-fructose eingesetzt. Diese Substanz wird aus reinen Saccharose-Lösungen durch enzymatische Umwandlung mit lebenden oder immobilisierten Zellsystemen nach bekannten Methoden (z. B. DBP 1 049 800) hergestellt.

Die α-D-Glucopyranosido-1,6-fructose wird in sauerstoffreiem Trinkwasser gelöst, das über Aktivkohle und Ionenfilter gereinigt wird.

Aus α-D-Glucopyranosido-1,6-fructose und entionisiertem Trinkwasser wird eine 45- bis 60 %ige, bevorzugt 50- bis 55 %ige wäßrige Lösung hergestellt, deren pH-Wert auf 3,5-6,5, bevorzugt 5-6,5 exakt eingestellt wird. Kristalline α-D-Glucopyranosido-1,6-fructose zeigt, gelöst in Wasser mit einem pH-Wert von 7, entweder einen neutralen oder — bedingt durch eine möglicherweise durch Cannizarro-Reaktion hervorgerufene spurenweise Gluconsäurebildung — eine schwach saure Reaktion. Die gewünschte pH-Wert-Einstellung kann z. B. durch Zugabe einer möglichst reinen organischen Säure erfolgen. Gut bewährt hat sich die Dotierung mit verdünnter Ameisensäure, Essigsäure, Zitronensäure, Sorbinsäure.

Für den Hydrierprozeß wird auf einen Druck von 100-500 bar, bevorzugt 200-300 bar, vorkomprimierter reiner Wasserstoff eingesetzt. Die Hydrierung erfolgt kontinuierlich im Festbettverfahren über als Hydrierkatalysatoren dienenden trägerfreien Formkörpern metallischer Art, indem man die zu hydrierende Lösung entweder im Gleichstrom mit dem zuvor zugemischten Wasserstoff von unten oder oben kommend über die in einen Hydrierreaktor gefüllten Formkörper strömen läßt oder auch von unten kommend dem von oben einströmmenden Wasserstoff entgegenführt oder umgekehrt (Gegenstromverfahren).

Der Hydrierreaktor kann entweder ein einzelnes Hochdruckrohr aus Stahl oder einer Stahllegierung sein, das mit den Formkörpern ganz oder teilweise gefüllt wird, wobei auch die Anwendung auf Horden (Drahtkörbe o. a.) nützlich sein kann, oder aber ein ummanteltes Hochdruckrohrbündel, dessen Einzelrohre mit Formkörpern ganz oder teilweise gefüllt werden.

Die trägerfreien Formkörper werden aus Metallpulvern von Elementen der 8. Nebengruppe des Periodensystems, insbesondere von Nickel, Kobalt und Eisen hergestellt, wobei entweder Pulver der reinen Metalle oder pulverisierte Legierungen der Metalle zum Einsatz kommen können. Die Herstellung der Formkörper erfolgt nach gebräuchlichen Methoden durch Verpressen der Metallpulver auf Tablettier- oder Pelletiermaschinen unter hohem Druck, wobei zur Verbesserung des Haftvermögens der Metallpartikel auch Graphit in kleinen Mengen oder Klebstoffe zum Einsatz kommen können. Die Herstellung der Formkörper muß in einer sauerstofffreien Atmosphäre erfolgen, um Oberflächenoxidationen zu vermeiden. Am wirksamsten und für die Reaktionsführung am günstigsten sind tablettierte oder pelletierte Formkörper mit Durchmessern von 5-10 mm. Von erheblicher Bedeutung ist die Druckfestigkeit der Formkörper, die erfindungsgemäß bei Werten zwischen 120-170 kg/cm$^2$ liegt. Niedrigere Druckfestigkeiten führen zu Formkörperzerfall bzw. erosivem Abrieb, was eine metallische Kontaminierung des Reaktionsproduktes bewirken würde. Von erheblicher Bedeutung ist weiterhin die innere Oberfläche der Formkörper, die erfindungsgemäß bei Werten zwischen 25 und 75 m$^2$/g liegt und ausschlaggebend für einen quantitativen Umsatz der Einsatzstoffe ist.

Der Hydrierprozeß wird bei einer Temperatur von 70 bis 115 °C, bevorzugt 80-110 °C durchgeführt. Niedrigere Temperaturen bedingen hohe Verweilzeiten oder Verzicht auf einen quantitativen Umsatz der α-D-Glucopyranosido-1,6-fructose. Höhere Temperaturen führen zu vermehrter Bildung von Zuckermonoalkoholen (Sorbit bzw. Mannit) sowie zu unkontrollierten Nebenreaktionen (Karamelisierung, hydrierende Crackung), was zu Verfärbungen sowie zur Bildung weiterer unerwünschter Nebenprodukte führen

kann. Die stündliche Katalysatorbelastung liegt zwischen 45 und 60 g α-D-Glucopyranosido-1,6-fructose/l Katalysator, bevorzugt 50-55 g/l. Bei exakter Einhaltung der Reaktionsbedingungen sind so ganz unerwartet hohe Katalysatorstandzeiten von 12.000 Stunden und mehr zu erzielen, was zu bisher bei der Hydrierung von α-D-Glucopyranosido-1,6-fructose noch nicht erreichten Katalysatorverbräuchen von $\leq$ 0,15 % führt. Damit liegen die hauptsächlichen technischen Vorteile des erfindungsgemäßen Verfahrens, außer in den durch den quantitativen Umsatz bedingten hohen Ausbeuten an α-D-Glucopyranosido-1,6-mannit und α-D-Glucopyranosido-1,6-sorbit und der Reinheit des Gemisches, die keine weiteren Reinigungsprozeduren notwendig macht, sowie der kostensparenden kontinuierlichen Arbeitsweise auch in dem extrem niedrigen Katalysatorverbrauch.

Die den Reaktor verlassende hydrierte wäßrige Lösung, die die beiden Zuckeralkohole α-D-Glucopyranosido-1,6-mannit und α-D-Glucopyranosido-1,6-sorbit im Verhältnis von ca. 1 : 1 enthält, wird nach der Entspannung, bei welcher man den überschüssigen Wasserstoff abfangen und nach erfolgter Komprimierung erneut zum Einsatz bringen kann, filtriert und kann so bereits direkt als Zuckeraustauschstoffmischung in flüssiger Form zum Einsatz kommen.

Das Wasser der Lösung läßt sich aber auch auf bekannte Weise über Sprühtrockner, Trockenwalzen oder Gefriertrocknung entfernen. Im Regelfall wird die nach dem Filtrieren erhaltene Farblose und glasklare Lösung in einem Fallfilmverdampfer oder einem ähnlich arbeitenden Gerät auf einen Zuckeralkoholgehalt von ca. 80 % aufkonzentriert und anschließend in einem Vakuum-Kristallisationsgerät zur vollständigen Kristallisation gebracht. Das Kristallisat läßt sich durch einen nachgeschalteten Mahlprozeß und eventuelle Siebung auf eine einheitliche Korngröße bringen. Obwohl das so gewonnene Produkt rieselfähig ist und völlig trocken erscheint, hat es einen Kristallwassergehalt von ca. 5 %, was darauf zurückzuführen ist, daß α-D-Glucopyranosido-1,6-mannit im Gegensatz zu α-D-Glucopyranosido-1,6-sorbit mit einem Gehalt von 10 % Kristallwasser kristallisiert.

Das gewonnene Produkt beginnt bei 90 °C zu schmelzen. Eine klare Schmelze bildet sich bei 140 °C. Den exakten Schmelzbereich des wasserfreien Substanzgemisches erhält man, wenn man z. B. das wasserhaltige Produkt in einem evakuierbaren Trockengerät bei 110 °C und 10 mbar zur Schmelze bringt und aus der Schmelze das Wasser quantitativ verdampfen läßt. Eine so behandelte rekristallisierte Probe zeigt ein Schmelzintervall von 138-143 °C.

In seinem Lösungsverhalten in Wasser liegt das 1 : 1-Gemisch von α-D-Glucopyranosido-1,6-mannit und α-D-Glucopyranosido-1,6-sorbit im Temperaturbereich von 0-70 °C zwischen dem der Reinsubstanzen. Bei Temperaturen über 70 °C übertrifft die Löslichkeit des Gemisches die der Reinsubstanzen (s. Löslichkeitsdiagramm), was den Einsatz der Mischung als Süßungsmittel für Getränke und Nahrungsmittel immer dann besonders vorteilhaft erscheinen läßt, wenn die infrage kommenden Stoffe stärker gesüßt werden sollen. Sowohl die Einzelverbindungen wie auch das Gemisch zeigen eine Süßkraft, die etwa 45 % der Süßkraft von Saccharose entspricht. Zur Erhöhung der Sußkraft des Gemisches von α-D-Glucopyranosido-1,6-mannit und α-D-Glucopyranosido-1,6-sorbit kann die wäßrige Lösung mit künstlichem Süßstoff, z. B. Cyclohexylsulfamat oder Phenylalaninasparaginsäuremethylester, versetzt und durch gemeinsame Vakuumkristallisation in kristalliner Form gewonnen werden. Man kann aber auch die Künstlichen Süßstoffe in fester Form mit dem Kristallisat vermischen. Das Gemisch von α-D-Glucopyranosido-1,6-mannit und α-D-Glucopyranosido-1,6-sorbit kann auch in flüssiger oder fester Form mit anderen süßschmeckenden Kohlenhydraten, z. B. Fructose, Sorbit, Xylit vermischt werden.

Das Gemisch von α-D-Glucopyranosido-1,6-mannit und α-D-Glucopyranosido-1,6-sorbit wird wie die Einzelstoffe von Hefen normalerweise nicht vergoren. Es wird auch nicht von handelsüblichen Invertasepräparaten und — Glucosidasen gespalten. Ebenso wie die Einzelstoffe ist daher auch die Mischung als süßschmeckender, kalorienarmer, struktur- und körperbildender Füllstoff ohne Bei- oder Nebengeschmack in Nahrungs- und Genußmitteln sowie in Getränken, die auch für Diabetiker geeignet und weniger kariogen sind als mit Zucker hergestellte Vergleichsprodukte, verwendbar.

## Beispiel 1

Ein senkrecht stehendes, wärmeisoliertes Hochdruckrohr aus nichtrostendem Stahl von 45 mm Innendurchmesser und 1 m Länge wird mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 147 kp/cm$^2$ und eine innere Oberfläche von 43 m$^2$/g aufweist. Durch dieses Rohr werden gemeinsam mit der dreifach molaren Menge von unter einem Druck von 300 bar stehendem hochreinen Wasserstoff stündlich 140 ml einer 50 %igen Lösung von α-D-Glucopyranosido-1,6-fructose in entionisiertem sauerstofffreien Trinkwasser, die auf einen pH-Wert von 6,0 eingestellt wurde, kontinuierlich gepumpt und zwar von unten nach oben aufsteigend.

Wäßrige Lösung und Wasserstoff werden durch einen Wärmeaustauscher geführt und so hoch erhitzt, daß sie in das Hochdruckrohr mit einer Temperatur von 80 °C eintreten. Das das Hochdruckrohr verlassende Gemisch von wäßriger Lösung und überschüssigem Wasserstoff wird über einen Kühler in einen Abscheider geführt, von wo der Wasserstoff nach Ersatz der verbrauchten Menge wieder zusammen mit noch nicht hydrierter Lösung in den Vorwärmer und von da erneut in das Hochdruckrohr gepumpt wird.

Die klare, wäßrige Lösung wird entspannt, über ein Feinfilter filtriert, in einem Fallfilmverdampfer auf

einen Zuckeralkoholgehalt von ca. 80 % aufkonzentriert und anschließend in einem Vakuumkristaller zur vollständigen Kristallisation gebracht. Das erhaltene feine Kristallpulver besteht aus einem Gemisch aus α-D-Glucopyranosido-1,6-mannit und α-D-Glucopyranosido-1,6-sorbit im Verhältnis von ca. 1 : 1 i.T. Der Wassergehalt liegt bei 5 %. Das Gemisch der beiden stereoisomeren Zuckeralkohole ist ansonsten hochrein (Reinheitsgrad ≥ 99,6 %). Der Gehalt an nicht hydrierter α-D-Glucopyranosido-1,6-fructose liegt bei ≤ 0,1 %. Der Gehalt an Sorbit liegt bei ≤ 0,1 %. Mannit konnte nicht nachgewiesen werden. Der Katalysator war auch nach einer Laufzeit von 12.000 Stunden unverändert wirksam. Das entspricht einem Katalysatorverbrauch < 0,15 %/kg hydrierte Substanz.

### Beispiel 2

Durch ein Hochdruckrohr, wie im Beispiel 1, wird bei einer Temperatur von 110 °C und einem Wasserstoffdruck von 200 bar im umgekehrten Reaktionsfluß, wie in Beispiel 1 beschrieben, stündlich eine gleichgroße Menge 50 %iger wäßriger Lösung von α-D-Glucopyranosido-1,6-fructose hydriert, die einen pH-Wert von 5,0 aufweist. Der Katalysator wurde durch Tablettierung von Nickelpulver hergestellt. Die Tabletten haben bei einer Zylinderhöhe von 5 mm und einem Durchmesser von 5 mm eine Druckfestigkeit von 143 kp/cm² und eine innere Oberfläche von 69 m²/g.

Nach einer Laufzeit von 12.000 Stunden mit unverminderter Wirksamkeit liegt der Gehalt an α-D-Glucopyranosido-1,6-mannit und α-D-Glucopyranosido-1,6-sorbit des in einem Rotationsverdampfer zur Trockne eingedampften Reaktionsgemisches bei 99,3 %. Der Gehalt an nicht hydrierter α-D-Glucopyranosido-1,6-fructose liegt bei 0,2 %. Der Gehalt an Sorbit liegt bei 0,1 %. Der Gehalt an Mannit beträgt 0,01 %.

### Beispiel 3

In einem Hochdruckrohr, wie in Beispiel 1, wird bei einer Temperatur von 115 °C und einem Wasserstoffdruck von 300 bar in gleicher Weise wie in Beispiel 1 stündlich eine gleichgroße Menge einer 50 %igen wäßrigen Lösung von α-D-Glucopyranosido-1,6-fructose hydriert, die einen pH-Wert von 5,5 aufweist. Der Katalysator wurde durch Tablettierung einer pulverisierten Nickel-Eisenlegierung gewonnen. Die Legierung enthält einen Eisenanteil in Nickel von 15 %. Die Tabletten haben bei einer Zylinderhöhe von 5 mm und einem Durchmesser von 5 mm eine Druckfestigkeit von 138 kg/cm² und eine innere Oberfläche von 63 m²/g. Das in einem Vakuumkristaller gewonnene 1 : 1-Gemisch von α-D-Glucopyranosido-1,6-mannit und α-D-Glucopyranosido-1,6-sorbit hat einen Reinheitsgrad von 99,3 %. Der Gehalt an nicht umgesetzter α-D-Glucopyranosido-1,6-fructose liegt bei 0,1 %. Der Sorbit-Gehalt beträgt 0,1 %. Der Mannitanteil beträgt 0,01 %. Der Katalysator war nach einer Laufzeit von 8.000 Stunden noch unverändert wirksam.

### Beispiel 4

In einem Hochdruckrohr, wie in Beispiel 1, wird bei einer Temperatur von 105 °C und einem Wasserstoffdruck von 200 bar in gleicher Weise wie in Beispiel 1 eine ebenso große Menge einer 50 %igen wäßrigen Lösung von α-D-Glucopyranosido-1,6-fructose hydriert, die einen pH-Wert von 6,0 aufweist. Der Katalysator wurde durch Tablettierung einer pulverisierten Nickel-Kobaltlegierung gewonnen. Die Legierung enthält einen Kobaltanteil in Nickel von 10 %. Die Tabletten haben bei einer Zylinderhöhe von 5 mm und einem Durchmesser von 5 mm eine Druckfestigkeit von 137 kg/cm² und eine innere Oberfläche von 29 m²/g. Das in einem Vakuum-Drehrohr gewonnene Gemisch von α-D-Glucopyranosido-1,6-mannit und α-D-Glucopyranosido-1,6-sorbit hat einen Reinheitsgrad von 99,2 %. Der Gehalt an nicht umgesetzter α-D-Glucopyranosido-1,6-fructose liegt bei 0,3 %. Der Sorbit-Gehalt beträgt 0,15 %. Mannit läßt sich nicht nachweisen. Der Katalysator war nach einer Laufzeit von 1.000 Stunden noch unvermindert wirksam.

### Beispiel 5 (Vergleichsbeispiel)

Durch ein Hochdruckrohr, wie in Beispiel 1, wird bei einer Temperatur von 100 °C und einem Wasserstoffdruck von 300 bar in gleicher Weise wie in Beispiel 1 in der gleichen Zeit eine ebenso große Menge einer 50 %igen wäßrigen Lösung von α-D-Glucopyranosido-1,6-fructose hydriert, die einen pH-Wert von 6,0 aufweist. Der Katalysator wurde durch Auftrag einer wäßrigen Nickelsalz-Lösung auf einen inerten kugelförmigen $Al_2O_3$-Träger (Kugeldurchmesser : 5 mm) und nachfolgende Überführung des Nickels in den metallischen Zustand durch Reduzierung im Wasserstoffstrom hergestellt. Der Nickelgehalt des Katalysators liegt bei 18 %. Die innere Oberfläche des Katalysators liegt bei 75 m²/g und entspricht somit der Oberfläche der vorbeschriebenen trägerfreien Katalysatoren. Das in einem Vakuumkristaller gewonnene 1 : 1-Gemisch von α-Glucopyranosido-1,6-mannit und α-D-Glucopyranosido-1,6-sorbit hat einen Reinheitsgrad von 91,9 %. Der Gehalt an nicht umgesetzter α-D-Glucopyranosido-1,6-fructose liegt bei 1,7 %. Der Sorbit- und Mannitgehalt liegt bei 0,3 %. Zusätzlich wurden unbekannte Verunreinigungen in einer Höhe von 6,1 % festgestellt, so daß das so erhaltene Gemisch von

α-D-Glucopyranosido-1,6-mannit und α-D-Glucopyranosido-1,6-sorbit in dieser Herstellungsform nicht als Zuckeraustauschstoff eingesetzt werden kann. Ferner wurde schon nach einer Laufzeit von 600 Stunden ein Nachlassen der Katalysatoraktivität beobachtet. Durch eine Erhöhung der Reaktionstemperatur von 100 auf 120 °C konnte zwar der Anteil an nicht umgesetzter α-D-Glucopyranosido-1,6-fructose auf einen Wert von 0,5 % gesenkt werden, gleichzeitig aber stieg der Anteil an unbekannten Verunreinigungen auf einen Wert von 6,4 % an.

Beispiel 6 (Vergleichsbeispiel)

Durch ein Hochdruckrohr, wie in Beispiel 1, wird bei einer Temperatur von 100 °C und einem Wasserstoffdruck von 300 bar in gleicher Weise wie in Beispiel 1 stündlich eine ebenso große Menge einer 50 %igen wäßrigen Lösung von α-D-Glucopyranosido-1,6-fructose hydriert, die einen pH-Wert von 6,0 aufweist. Der Katalysator wurde durch Auftrag von wäßrigen Nickel- und Eisensalz-Lösungen auf einen inerten, kugelförmigen $Al_2O_3$-Träger (Kugeldurchmesser : 5 mm) und nachfolgende Überführung des Nickels und Eisens in den metallischen Zustand durch Reduzierung im Wasserstoffstrom hergestellt. Der Nickelgehalt des Katalysators liegt bei 16 %, der Eisengehalt bei 4 %. Die innere Oberfläche des Katalysators liegt bei 75 $m^2$/g. Das durch Eindampfen in einem Vakuumkristaller gewonnene 1 :1-Gemisch von α-D-Glucopyranosido-1,6-mannit und α-D-Glucopyranosido-1,6-sorbit hat einen Reinheitsgrad von 93,2 %. Der Gehalt an nicht umgesetzter α-D-Glucopyranosido-1,6-fructose liegt bei 1,5 %. Der Sorbit- und Mannitgehalt liegt bei 0,2 %. Zusätzlich wurden unbekannte Verunreinigungen in einer Höhe von 5,1 % festgestellt, so daß das so erhaltene Gemisch von α-D-Glucopyranosido-1,6-mannit und α-D-Glucopyranosido-1,6-sorbit nicht direkt als Zuckeraustauschstoff eingesetzt werden kann. Schon nach einer Laufzeit von 800 Stunden wurde ein erhebliches Nachlassen der Katalysatoraktivität festgestellt. Der Katalysatorverbrauch lag bei > 1,5 %/kg hydrierte Substanz.

**Patentansprüche**

1. Verfahren zur Herstellung eines Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100-500 bar und Temperaturen von 70-115 °C im Festbettverfahren über als Hydrierungskatalysatoren dienenden trägerfreien Formkörpern mit einer Druckfestigkeit von 120-170 kg/cm2 und einer inneren Oberfläche zwischen 25 und 75 $m^2$/g von Elementen der 8. Nebengruppe des Periodensystems durchführt.

2. Verfahren nach Anspruch 1, dadurch gekennzeichnet, daß es sich bei den als Hydrierungskatalysatoren dienenden trägerfreien Formkörpern um aus Metallpulvern aus Nickel, Kobalt, Eisen hergestellte Formkörper handelt.

3. Verfahren nach Anspruch 1, dadurch gekennzeichnet daß es sich bei den als Hydrierungskatalysatoren dienenden trägerfreien Formkörpern um aus Pulvern der reinen Metalle oder aus Legierungen dieser Metalle hergestellte Formkörper handelt.

4. Verfahren nach Anspruch 1, dadurch gekennzeichnet, daß man die Hydrierung der α-D-Glucopyranosido-1,6-fructose in 45- bis 60 %iger wäßriger Lösung unter einem pH-Wert von 3,5-6,5 durchführt.

**Claims**

1. Process for the preparation of a mixture of α-D-glucopyranosido-1,6-mannitol and α-D-glucopyranosido-1,6-sorbitol from α-D-glucopyranosido-1,6-fructose by catalytic hydrogenation in aqueous solution with hydrogen under increased pressure and at elevated temperature, characterized in that the hydrogenation is carried out continuously at a hydrogen pressure of 100-500 bar and temperatures of 70-115 °C in a fixed bed process over support-free shaped pieces of elements of subgroup 8 of the periodic table, which serve as hydrogenation catalysts and have a compressive strength of 120-170 kg/$cm^2$ and an internal surface area of between 25 and 75 $m^2$/g.

2. Process according to Claim 1, characterized in that the support-free shaped pieces which serve as hydrogenation catalysts are shaped pieces prepared from metal powders consisting of nickel, cobalt or iron.

3. Process according to Claim 1, characterized in that the support-free shaped pieces which serve as hydrogenation catalysts are shaped pieces prepared from powders of the pure metals or from alloys of these metals.

4. Process according to Claim 1, characterized in that the hydrogenation of the α-D-glucopyranosido-1,6-fructose is carried out in 45 to 60 % strength aqueous solution at a pH of 3.5-6.5.

**Revendications**

1. Procédé pour la fabrication d'un mélange d'α-D-glucopyrannosido-1,6-mannitol et d'α-D-glucopyrannosido-1,6-sorbitol à partir d'α-D-glucopyrannosido-1,6-fructose par hydrogénation catalytique en solution aqueuse par l'hydrogène sous pression élevée et à température élevée, caractérisé en ce que l'on met en œuvre l'hydrogénation en continu sous une pression d'hydrogène de 100-500 bar et à des température de 70-115 °C dans un procédé à lit fixe sur des corps moulés sans support d'éléments du huitième sous-groupe de la classification périodique des éléments servant de catalyseurs d'hydrogénation, d'une résistance à la pression de 120-170 $kg/cm^2$ et d'une surface interne comprise entre 25 à 75 $m^2/g$.

2. Procédé selon la revendication 1, caractérisé en ce que les corps moulés sans support servant de catalyseurs d'hydrogénation sont des corps moulés fabriqués à partir de poudres de nickel, cobalt, fer.

3. Procédé selon la revendication 1, caractérisé en ce que les corps moulés sans support servant de catalyseurs d'hydrogénation sont des corps moulés fabriqués à partir de poudres des métaux purs ou d'alliages de ces métaux.

4. Procédé selon la revendication 1, caractérisé en ce que l'on met en œuvre l'hydrogénation de l'α-D-glucopyrannosido-1,6-fructose en solution aqueuse à 45-60 % à un pH de 3,5-6,5.